(19) Europäisches Patentamt
European Patent Office
Office européen des brevets

(11) Numéro de publication: **0 381 570 A1**

(12) **DEMANDE DE BREVET EUROPEEN**

(21) Numéro de dépôt: **90400242.5**

(22) Date de dépôt: **29.01.90**

(51) Int. Cl.5: **C07D 281/10, //C07B57/00, C07C323/56**

(30) Priorité: **30.01.89 FR 8901131**

(43) Date de publication de la demande: **08.08.90 Bulletin 90/32**

(84) Etats contractants désignés: **AT BE CH DE DK ES FR GB GR IT LI LU NL SE**

(71) Demandeur: **SANOFI**
**40, Avenue George V**
**F-75008 Paris(FR)**

(72) Inventeur: **Bousquet, André**
**Avenue du Lac**
**F-04200 Sisteron(FR)**
Inventeur: **Charpiot, Brigitte**
**Place du Jeu de Paume - Ribiers**
**F-05300 Laragne(FR)**
Inventeur: **Heymes, Alain**
**Rue Frédéric Mistral**
**F-04200 Sisteron(FR)**

(74) Mandataire: **Le Guen, Gérard et al**
**CABINET LAVOIX 2, place d'Estienne d'Orves**
**F-75441 Paris Cédex 09(FR)**

(54) **Procédé de préparation de dérivés de benzothiazépine-1,5 one-4.**

(57) L'invention a pour objet un procédé de préparation de dérivés de benzothiazépine- 1,5 one-4 de formule générale

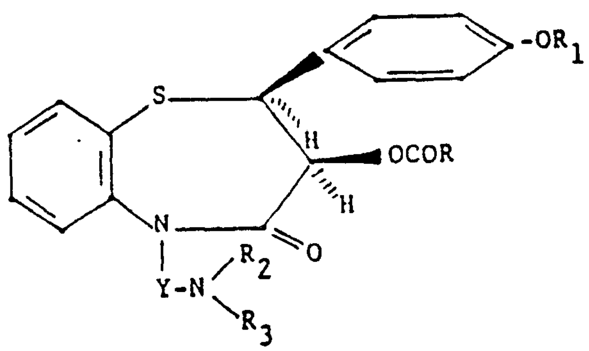

I

dans laquelle R, $R_1$, $R_2$ et $R_3$, identiques ou différents, représentent chacun un radical alkyle en $C_1$-$C_4$ et Y représente un radical alkylène en $C_1$-$C_4$ ainsi que leurs sels pharmaceutiquement acceptables, comprenant les étapes de :
- résolution de sels optiquement actifs
- cyclisation
- alkylation en présence d'hydroxyde, carbonate ou hydrogénocarbonate de potassium dans un solvant choisi parmi le formamide, l'acétamide, le N,N-diméthylformamide et le N,N-diméthylacétamide
- acylation.

EP 0 381 570 A1

La présente invention se rapporte, d'une manière générale, à un nouveau procédé de préparation de dérivés de benzothiazépine-1,5 one-4.

En particulier, l'invention concerne un procédé pour préparer les dérivés de benzothiazépine-1,5 one-4 de formule générale :

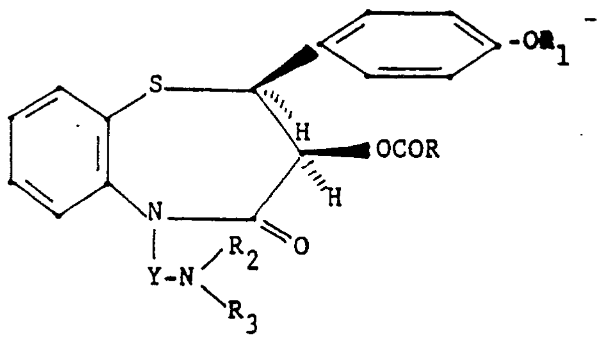

I

dans laquelle R, $R_1$, $R_2$ et $R_3$, identiques ou différents, représentent chacun un radical alkyle en $C_1$-$C_4$ et Y représente un radical alkylène en $C_1$-$C_4$ ainsi que leurs sels pharmaceutiquement acceptables.

Les composés de formule I ci-dessus se présentent sous la forme d'isomères cis (2S, 3S) et sont connus comme possédant des propriétés antagonistes de la translocation calciques, hypotensives et vasodilatatrices coranariennes et cérébrales.

A ce titre, les composés de formule I peuvent être utilisés dans le traitement de l'hypertension, de l'angine de poitrine et des arythmies cardiaques.

De ce point de vue, le composé de formule I le plus intéressant est celui dans lequel R, $R_1$, $R_2$ et $R_3$ représentent chacun le radical méthyle et Y représente le radical éthylène, c'est-à-dire la cis(+)(2S, 3S) acétoxy-3(N,N-diméthylamino-2 éthyl)-5 (méthoxy-4 phényl)-2 dihydro-2,3 benzothiazépine (5H)-1,5 one-4 appelée communément diltiazem.

En conséquence, la présente invention se rapporte en particulier à un procédé pour la préparation du diltiazem et de ses sels pharmaceutiquement acceptables.

Ces composés de formule I sous forme racémique (+/-)(2S, 3S) ou sous forme d'isomères séparés ont été décrits par exemple dans le brevet français No 2.530.243.

On connaît déjà des procédés permettant la préparation des dérivés de benzothiazépine-1,5 one-4 de formule I. Ces composés étant caractérisés par une isomérie cis (2S, 3S), il est nécessaire, à un stade quelconque de leur synthèse, de sélectionner la configuration appropriée. Cette résolution d'isomères optiques peut s'effectuer au stade final de la préparation mais plus généralement au niveau du mélange racémique d'un des intermédiaires de synthèse. En effet, le composé final de formule I peut être obtenu à partir d'un composé intermédiaire présentant les caractéristiques isomériques appropriées sans racémisation au cours des étapes ultérieures.

Parmi les procédés utilisant un tel schéma, on peut citer le procédé décrit dans la demande de brevet japonais No 78-18038 qui comporte la suite des 5 étapes suivantes au départ de l'étape de résolution des isomères optiques d'un des intermédiaires de synthèse, à savoir :

- résolution d'un acide (+/-)thréo hydroxy-2 (alkoxy-4 phényl)-3 (nitro-2 phénylthio)-3 propionique par passage au sel formé avec la cinchonidine, l'éphédrine ou la quinine
- réduction de l'acide cis(+) hydroxy-2(alkoxy-4 phényl)-3 (nitro-2 phénylthio)-3 propionique obtenu, avec l'hydrogène, en présence de charbon palladié, dans l'acide acétique
- cyclisation de l'acide cis(+) hydroxy-2-(alkoxy-4 phényl)-3 (amino-2 phénylthio)-3 propionique dans le xylène au reflux pendant 12 heures
- condensation de la cis(+)(2S, 3S) hydroxy-3 (alkoxy-4 phényl)-2 dihydro-2,3 benzothiazépine (5H)-1,5 one-4 obtenue avec le chlorure de diméthylaminoéthyle en présence d'hydrure de sodium, de sodium métallique ou d'un amidure de sodium
- acétylation de la cis(+)(2S, 3S) hydroxy-3(N,N-diméthylamino-2 éthyl)-5 (alkoxy-4 phényl)-2 dihydro-2,3 benzothiazépine (5H)-1,5 one-4 avec l'acide acétique.

Cependant, aucun exemple n'illustre la mise en oeuvre de ce proccédé et aucun rendement global ne permet de juger de son efficacité.

De même, on peut mentionner le procédé exemplifié dans le brevet français NO 2.530:243 lequel comporte les 4 étapes suivantes y compris l'étape de résolution des isomères optiques d'un des

intermédiaires de synthèse, à savoir :

- résolution de l'acide (+/-)thréo hydroxy-2 (alkoxy-4 phényl)-3 (amino-2 phénylthio)-3 propionique par passage au sel formé avec la d-α-phényléthylamine,
- salification de l'acide (+)thréo hydroxy-2 (alkoxy-4 phényl)-3 (amino-2 phénylthio)-3 propionique avec la pyridine,
- cyclisation/acétylation du sel formé, avec de 2 à 2,5 équivalents d'anhydride acétique,
- alkylation avec le chlorure de diméthyl aminoéthyle en présence d'hydrure de sodium et de gel de silice dans le diméthylsulofxyde.

Appliqué à la préparation du diltiazem, ce procédé a fourni un rendement molaire de 47% en produit désiré.

Les procédés antérieurs décrits ci-dessus ne sont toutefois pas sans présenter plusieurs inconvénients limitant leur utilisation lorsqu'ils sont appliqués au niveau industriel.

On sait par exemple que la mise en oeuvre de métaux alcalins, de même que leurs hydrure et amidure, peut être dangereuse dans la plupart des cas.

En outre, l'utilisation d'hydrure de sodium dans le diméhylsulfoxyde présente de graves inconvénients au niveau de la sécurité étant donné les risques d'explosions enregistrés avec ce système. De plus, l'emploi d'hydrure de sodium et de diméthylsulfoxyde s'accompagne inévitablement de réactions secondaires dues au carbanion méthylsufinyle ($CH_3SOCH_2^-$).

Par ailleurs, la demande de brevet européen $A_1$-0 081 234 décrit un procédé de N-alkylation d'une benzothiazépine effectuée en présence d hydroxyde ou de carbonate de potassium.

Ce procédé met nettement en évidence le rôle du solvant pour l'obtention de rendements importants en benzothiazépine N-alkylée.

La N-alkylation en question est effectuée en présence d'hydroxyde ou de carbonate de potassium dans l'acétone, un acétate d'alkyle ou une solution aqueuse de ces solvants, ce qui permet des rendements de l'ordre de 90% en composé N-alkylé.

Toutefois, la même réaction de N-alkylation pratiquée dans d'autres solvants tels que le méthanol, le dioxanne ou le toluène, a fourni des rendements nuls ou de 30% maximum en composé désiré.

On a maintenant trouvé qu'il est possible de préparer les dérivés de benzothiazépine de formule I selon un procédé en 4 étapes parfaitement utilisable au niveau industriel et comportant comme première étape la résolution des isomères optiques d'un composé intermédiaire, et une étape de N-alkylation effectuée en présence d'hydroxyde, de carbonate ou d'hydrogénocarbonate de potassium.

On a découvert de façon surprenante que la réaction de N-alkylation pouvait être effectuée dans des solvants autres que l'acétone, les acétates d'alkyle ou les solutions aqueuses de ces solvants avec des rendements très importants.

Les solvants utilisés dans le procédé selon l'invention sont le formamide, l'acétamide, le N,N-diméthylformamide et le N,N-diméthylacétamide.

On a par exemple trouvé selon les cas des rendements en benzothiazépine N-alkylée supérieurs à 90% lorsque le solvant utilisé est le N,N-diméthylformamide.

Ainsi, le procédé de l'invention permettant la préparation des dérivés de formule I s'effectue selon le déroulement d'étapes suivantes selon lesquelles :

1 - résolution

on sépare un dérivé d'acide (+/-)thréo hydroxy-2 phényl-3 (amino-2 phénylthio)-3 propionique de formule générale :

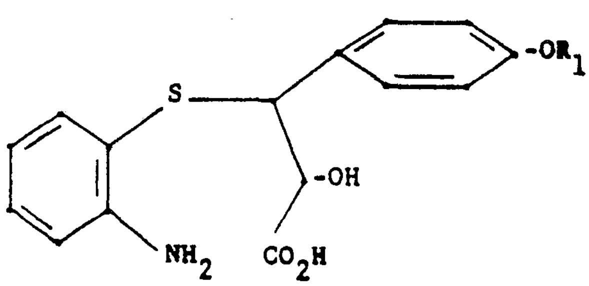

II

dans laquelle $R_1$ a la même signification que précédemment, sous forme de sel avec une base optiquement active pour obtenir l'acide thréo hydroxy-2 phényl-3 (amino-2 phénylthio)-3 propionique correspondant,

2 - cyclisation

on cyclise par chauffage à une température allant de 100 à 140° C dans un hydrocarbure aromatique choisi parmi le toluène, un xylène et l'éthylbenzène; l'acide thréo hydroxy-2 phényl-3 (amino-2 phénylthio)-3 propionique ainsi obtenu, ce qui fournit un dérivé cis(2S, 3S) de formule générale :

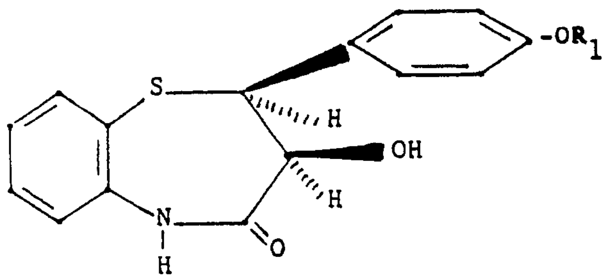

III

dans laquelle $R_1$ a la même signification que précédemment,

3 - alkylation

on fait réagir le dérivé cis(2S, 3S) de formule III avec un halogénure, de préférence le chlorure, de formule générale :

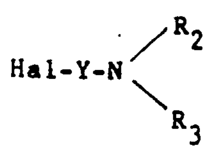

IV

ou un sel de cet halogénure, par exemple le chlorhydrate, dans laquelle Hal, Y, $R_2$ et $R_3$ ont la même signification que précédemment, en présence d'un agent basique choisi parmi l'hydroxyde, le carbonate ou l'hydrogénocarbonate de potassium et dans un solvant choisi parmi le formamide, l'acétamide, le N,N-diméthylformamide et le N,N-diméthylacétamide, pour obtenir un dérivé cis(2S, 3S) de benzothiazépine- 1,5 one-4 de formule générale :

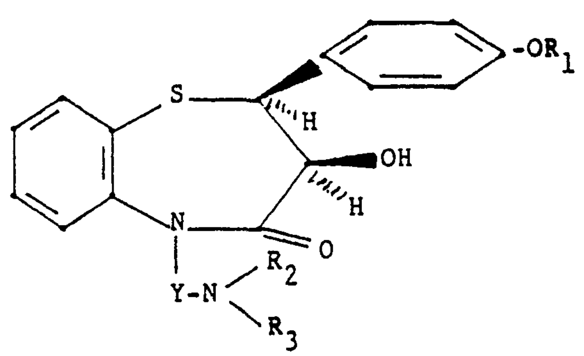

V

dans laquelle $R_1$, $R_2$, $R_3$ et Y ont la même signification que précédemment,

4 - acylation

on fait réagir, dans un hydrocarbure aromatique au reflux, le dérivé cis(2S, 3S) de benzothiazépine-1,5 one-4 de formule V, avec un agent d'acylation approprié, à savoir un anhydride de formule générale

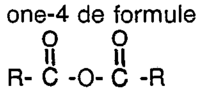 VI

dans laquelle R a la même signification que précédemment, pour obtenir le composé désiré de formule I sous forme de base libre que l'on peut faire réagir, si nécessaire, avec un acide organique ou inorganique approprié pour former un sel pharmaceutiquement acceptable.

Pour séparer les acides de formule II on peut procéder de la façon suivante : on soumet l'acide de formule II à une réaction de salification dans un solvant, avec la d-α-phényléthylamine, on sépare les deux sels optiquements actifs par cristallisation fractionnée et on traite le sel du dérivé d'acide thréohydroxy-2 phényl-3 (amino-2 phénylthio)-3 propionique de formule II et de la d-α-phényléthylamine avec un acide organique ou inorganique.

A cet effet on peut faire réagir avec l'acide de formule II 1 à 1,2 équivalent de d-α-phényléthylamine dans un solvant approprié, par exemple l'eau, un solvant organique polaire tel que le méthanol, l'éthanol, l'acétone, la méthyléthylcétone ou une solution aqueuse de ces solvants. On préfère toutefois utiliser l'eau pour cette opération. On isole alors par filtration, le sel du dérivé d'acide thréo propionique qui a précipité puis on régénère le dérivé d'acide thréo propionique par hydrolyse d'une solution aqueuse du sel du dérivé d'acide thréo propionique en question selon des procédés connus par exemple par acidification au moyen d'un acide tel que l'acide chlorhydrique.

On cyclise alors le dérivé d'acide thréo propionique de préférence dans un xylène au reflux, ce qui nécessite de 7 à 10 heures, et on fait réagir les composés de formules III et IV dans des proportions sensiblement stoechiométriques, préférentiellement en présence de carbonate de potassium que l'on utilise généralement à raison de 1 à 3 moles par mole de composé de formule III. Cette réaction est effectuée à une température allant de la température ambiante à 80 °C, de préférence à une température allant de 50 à 70 °C et dans un solvant choisi parmi : le formamide, l'acétamide, le N,N-diméthylformamide et le N,N-diméthylacétamide. On préfère cependant le N,N-diméthylformamide comme solvant.

Quant à la réaction du composé de formule V avec l'anhydride de formule VI, celle-ci s'effectue dans des proportions sensiblement stoechiométriques habituellement dans un hydrocarbure aromatique tel que par exemple le toluène.

Les dérivés d'acide (+/-)thréo propionique de formule II sont des produits connus et peuvent être préparés par application de la méthode décrite dans le brevet français No 2.530.243 ou par application de la succession d'étapes suivantes :

a) on fait réagir une benzaldéhyde de formule générale :

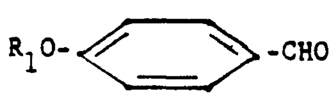 VII

dans laquelle $R_1$ a la même signification que précédemment, avec un halogénoacétate d'alkyle de formule générale :

$Hal-CH_2-CO_2R_4$

dans laquelle $R_4$ représente un radical alkyle en $C_1$-$C_4$, pour obtenir les esters glycidiques trans(2RS, 3SR) de formule générale :

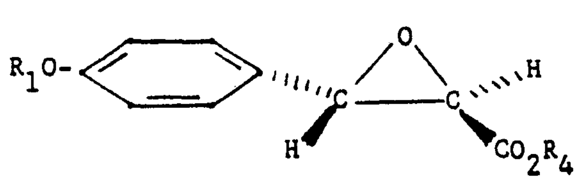 VIII

dans laquelle $R_1$ et $R_4$ ont la même signification que précédemment,

b) on fait réagir le dérivé de formule VIII avec l'amino-2 thiophénol pour donner les composés thréo-(2RS, 3SR) de formule générale :

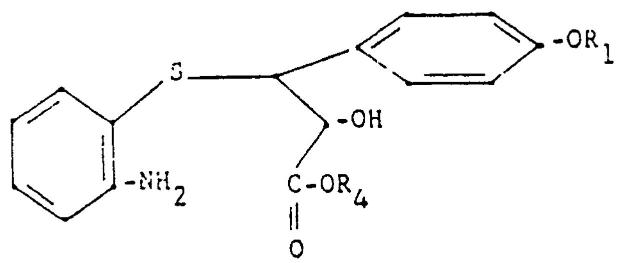

IX

dans laquelle $R_1$ et $R_4$ ont la même signification que précédemment,

c) on saponifie le composé de formule IX au moyen d'un agent basique tel qu'un hydroxyde de métal alcalin par exemple l'hydroxyde de sodium ou un carbonate de métal alcalin par exemple le carbonate de potassium, et on forme après acidification les acides thréo(2RS, 3SR) propioniques de formule II.

Les composés de formules VII sont des composés connus et peuvent être préparés par des méthodes connues.

Le procédé de l'invention ainsi décrit permet l'obtention de rendements molaires importants en dérivés de benzothiazépine-1,5 one-4 pouvant s'avérer, dans certains cas, de 25% supérieurs aux rendements enregistrés avec le procédé du brevet français No 2.530.243.

Par exemple, on a pu obtenir jusqu'à 60% de rendement molaire en chlorhydrate de diltiazem à partir de l'acide racémique de formule II alors que le procédé de l'art antérieur n'en a fourni que 47%.

En outre, le procédé de l'invention évite l'utilisation d'hydrure de sodium, d'amidure de sodium ou de sodium métallique ainsi que l'emploi du couple hydrure de sodium/diméthylsulfoxyde dans l'étape de N-alkylation. De même, le procédé de l'invention ne nécessite pas, comme dans le procédé antérieur, l'usage de gel de silice qu'il importe d'éliminer augmentant de ce fait le nombre de manipulations.

L'exemple non limitatif suivant illustre le procédé de l'invention :

## EXEMPLE

Préparation du chlorhydrate de cis(+)(2S,3S) acétoxy-3 (N,N-diméthylamino-2 éthyl)-5 (méthoxy-4 phényl)-2 dihydro-2,3 benzothiazépine (5H)-1,5 one-2

a) Epoxy-2,3 (méthoxy-4 phényl)-3 propionate de méthyle (isomère E)

Dans un réacteur de 500 ml équipé d'un système de chauffage-refroidissement, d'un thermomètre, d'un agitateur mécanique, d'un réfrigérant et d'une ampoule d'introduction, on place, sous atmosphère inerte, 250 ml de méthanol absolu et 17,3g (0,75 mole) de sodium en lamelles. Lorsque le sodium est entièrement consommé, on refroidit le milieu réactionnel à -10° C et on ajoute en environ 2,5 heures un mélange de 68g (0,5 mole) de p-anisaldéhyde et 81,4g (0,75 mole) de chloroacétate de méthyle. En fin d'addition on laisse la température du milieu réactionnel s'élever jusqu'à 0° C pendant une heure puis jusqu'à 20° C durant une heure. On refroidit alos le milieu réactionnel et on le verse dans 300ml d'eau. On observe une précipitation du produit attendu que l'on filtre et lave à l'eau. Après séchage à l'étuve, on isole 92g d'un produit brut (rendement massique : 90%).

De cette manière, on obtient l'époxy-2,3 (méthoxy-4 phényl)-3 propionate de méthyle (isomère E) que l'on purifie par recristallisation dans l'éther diisopropylique de manière à obtenir un composé analytiquement pur. P.F. : 69° C.

b) Thréo(+/-) hydroxy-2 (amino-2 phénylthio)-3 (méthoxy-4 phényl)-3 propionate de méthyle.

Dans un réacteur de 250 ml muni d'un système de chaufage-refroidissement, d'un agitateur mécanique, d'un thermomètre et d'un réfrigérant, on place, sous atmosphère inerte, 13,875g (0,11 mole) d'O-aminothiophénol, 20,8g (0,1 mole) d'époxy-2,3 (méthoxy-4 phényl)-3 propionate de méthyle et 100ml de

toluène. On porte l'ensemble au reflux durant 2 heures puis on distille le toluène de manière à obtenir 34g d'un résidu solide (rendement massique : 100%).

De cette manière, on obtient le thréo(+/-) hydroxy-2 (amino-2 phénylthio)-3 (méthoxy-4 phényl)-3 propionate de méthyle qui peut être utilisé à l'état brut dans l'étape suivante ou recristallisé dans un mélange méthanol/eau de manière à obtenir un composé analytiquement pur.

c) Acide (+/-)thréo hydroxy-2 phénylthio)-3 (méthoxy-4 phényl)-3 propionique.

Dans un réacteur de 250ml muni d'un système de chauffage-refroidissement, d'un agitateur mécanique, d'un thermomètre et d un réfrigérant, on place, sous atmosphère inerte 33,3g (0,1 mole) de thréo (+/-) hydroxy-2 (amino-2 phénylthio)-3 (méthoxy-4 phényl)-3 propionate de méthyle brut et 160ml d'hydroxyde de sodium à 5%. Sous agitation vive, on porte le milieu à 50° C et on l'y maintient durant 2 heures. Le milieu réactionnel est limpide et brun. Après refroidissement à température ambiante, on verse le mélange dans 82ml d'acide chlorhydrique à 9%, ce qui provoque la précipitation du produit attendu. On le filtre et on le lave à l'eau puis on le sèche à l'étuve sous vide à 20° C.

De cette manière, on obtient 31,5g d'acide (+/-)thréo hydroxy-2 (amino-2 phénylthio)-3 (méthoxy-4 phényl)-3 propionique sous forme brute (rendement massique : 100%) que l'on peut purifier par réempâtage dans l'éthanol. P.F. du produit pur : 169° C.

d) Acide (+) thréo hydroxy-2 (méthoxy-4 phényl)-3 (aminno-2 phénylthio)-3 propionique.

Dans un réacteur de 61ml muni d'un système de chauffage-refroidissement, d'un agitateur mécanique, d'un thermomètre et d'un réfrigérant, on introduit, sous atmosphère inerte, 249,1g (0,78 mole) d'acide (+/-)-thréo hydroxy-2 (amino-2 phénylthio)-3 (méthoxy-4 phényl)-3 propionique purifié et 3900 ml d'eau. On chauffe le milieu à 50° C et on additionne, progressivement, 100,7g (0,830 mole) de d-α-phényléthylamine. On chauffe jusqu'à homogénéisation du milieu puis on le refroidit sous agitation, à température ambiante. On observe alors une précipitation du sel d'amine que l'on filtre puis redissout par chauffage dans 1260ml d'eau. En refroidissant le milieu réactionnel à température ambiante, on peut isoler un sel pur de l'acide (+)thréo hydroxy-2 (méthoxy-4 phényl)-3 (amino-2 phénylthio)-3 propionique et de la d-α-phényléthylamine. On met ce sel en suspension dans 500 ml d'eau et on y ajoute 300ml d'une solution d'hydroxyde de sodium 1N tout en maintenant le milieu à température ambiante. On extrait avec du dichlorométhane puis on verse la phase aqueuse dans 1600ml d'acide chlorhydrique 0,18N. après ajustement du pH à 3,5 l'acide attendu précipite. On le filtre, on le lave à l'eau et on le sèche à l'étuve à vide à 50° C.

De cette manière, on isole 95g d'acide (+)thréo hydroxy-2 (méthoxy-4 phényl)-3 (amino-2 phénylthio)-3 propionique.

Rendement massique : 38% (à partir de l'acide racémique) soit un rendement molaire de 76%. P.F. : 138° C, $\alpha_D^{20}$ = 346°

e) Cis(+) (2S,3S)hydroxy-3 (méthoxy-4 phényl)-2 dihydroxy-2,3 benzothiazépine (5H)-1,5 one-4.

Dans un réacteur de 21ml muni d'un système de chauffage-refroidissement, d'un agitateur mécanique, d'un thermomètre, d'un réfrigérant et d'un système Dean Stark, on introduit, sous atmosphère inerte, 50g (0,156 mole) d'acide (+)thréo hydroxy-2 (méthoxy-4 phényl)-3 (amino-2 phénylthio)-3 propionique et 940ml de xylène. On porte l'ensemble au reflux et on l'y maintient durant 8 heures. On refroidit à -10° C, on filtre, on rince le solide avec du xylène et on sèche en étuve ventilée. De cette manière, on obtient 43g de cis(+) (2S,3S) hydroxy-3 méthoxy-4 phényl)-2 dihydro-2,3 benzothiazépine (5H)- 1,5 one-4. Rendement : 91%, P.F. 200° C, $\alpha_D^{20}$ = + 115.

Le filtrat contient encore 6 à 7% du produit désiré que l'on peut récupérer.

f) Cis(+)(2S,3S)hydroxy-3 (N,N-diméthylamino-2 éthyl)-5 (méthoxy-4 phényl)-2 dihydro-2,3 benzothiazépine (5H)-1,5 one-4.

Dans un réacteur de 250ml d'un système de chauffage-refroidissement, d'un agitateur mécanique, d'un thermomètre et d'un réfrigérant, on introduit, sous atmosphère inerte, 12g (0,04 mole) de cis(+) (2S,3S)

hydroxy-3 (méthoxy-4 phényl)-2 dihydro-2,3 benzothiazépine (5H)-1,5 one-4 et 60ml de N,N-diméthylformamide anhydre. Le milieu étant sous agitation, on obtient une solution limpide. On additionne alors 16,6g (0,12 mole) de carbonate de potassium et 6,9g (0,048 mole) de chlorhydrate de diméthylamino-2 chloro-1 éthane. On chauffe l'ensemble à 60° C durant une heure. Après refroidissement du milieu à température ambiante, on filtre les sels minéraux et on concentre sous vide. On reprend dans 75ml de dichloroéthane le résidu huileux obtenu et on lave à l'eau. On concentre la phase organique et on reprend le résidu dans l'éther de pétrole de manière à obtenir la cristallisation du produit attendu. On filtre et on sèche à l'étuve sous vide.

De cette manière, on obtient 13,65g de cis(+)(2S,3S) hydroxy-3 (N,N-diméthylaminoéthyl)-5 (méthoxy-4 phényl)-2 dihydro-2,3 benzothiazépine (5H)-1,5 one-4. Rendement : 91,6%, P.F. : 86-87 °C, $\alpha_D^{20}$ = + 168,8° (c = 0,25, méthanol)

g) Chlorhydrate de cis(+)(2S,3S) acétoxy-3 (N,N-diméthylamino-2 éthyl)-5 (méthoxy-4 phényl)-2 dihydro-2,3 benzothiazépine (5H)- 1,5 one-2.

Dans un ballon tricol de 50ml muni d'un système de chauffage-refroidissement, d'un agitateur mécanique, d'un thermomètre et d'un réfrigérant, on introduit 5,6g (0,015 mole) de cis(+)(2S,3S) hydroxy-3 (N,N-diméthylamino-2 éthyl)-5 (méthoxy-4 phényl)-2 dihydro-2,3 benzothiazépine (5H)-1,5 one-2 et 14ml de toluène sec. On place l'ensemble sous agitation et on additionne 1,7g (0,0165 mole) d'anhydride acétique. On chauffe le milieu au reflux durant 3 heures puis on refroidit à température ambiante et on ajoute 3,4ml d'une solution éthanolique d'acide chlorhydrique 4,8N. On refroidit à 0° C et on filtre le précipité obtenu. On lave au toluène et on sèche.

De cette manière, on obtient 6,25g de chlorhydrate de cis(+)(2S,3S) acétoxy-3 (N,N-diméthylamino-2 éthyl)-5 (méthoxy-4 phényl)-2 dihydro-2,3 benzothiazépine (5H)- 1,5 one-4. Rendement 92,4%, P.F. : 210° C, $\alpha_D^{20}$ = + 116,5° (c = 1%, eau).

**Revendications**

1. Procédé de préparation de dérivés de benzothiazépine-1,5 one-4 de formule générale

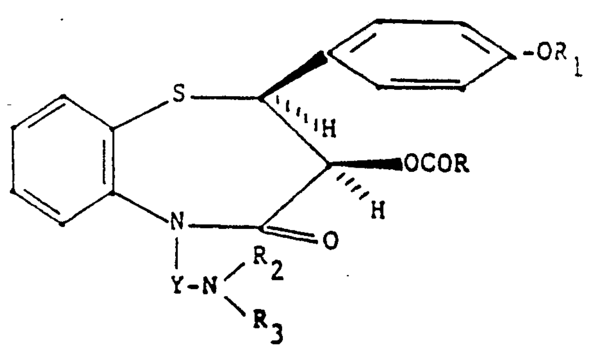

I

dans laquelle R, $R_1$, $R_2$ et $R_3$, identiques ou différents, représentent chacun un radical alkyle en $C_1$-$C_4$ et Y représente un radical alkylène en $C_1$-$C_4$ ainsi que leurs sels pharmaceutiquement acceptables, comprenant les étapes suivantes selon lesquelles :

a) on fait réagir un ester glycidique trans (2RS, 3SR) de formule générale :

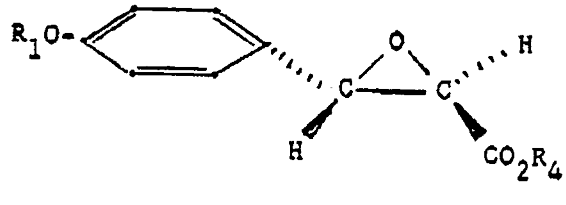

dans laquelle $R_1$ a la même signification que précédemment et $R_4$ représente un radical alkyle en $C_1$-$C_4$, avec l'amino-2 thiophénol pour donner les composés threo (2 S, 3 SR) de formule générale :

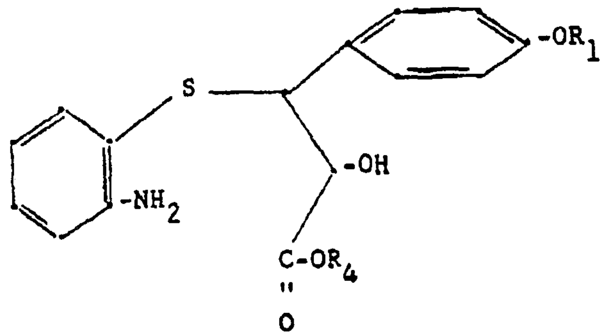

IX

dans laquelle $R_1$ et $R_4$ ont la même signification que précédemment,

b) on saponifie le composé de formule IX au moyen d'un agent basique et on forme après acidification les acides (+) et (-) hydroxy -2(alkoxy-4 phényl)-3(amino-2 phénylthio)-3 propioniques de formule générale :

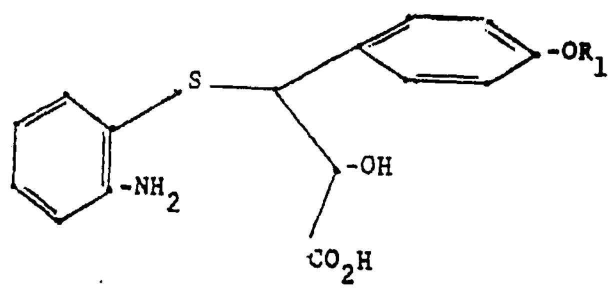

dans laquelle $R_1$ a la même signification que précédemment,

c) on sépare les acides (+) et (-)hydroxy-2 (alkoxy-4 phényl)-3 (amino-2 phénylthio)-3 propioniques de formule II sous forme de sel avec une base optiquement active, pour obtenir l'acide thréo hydroxy-2 (alkoxy-4 phényl)-3 (amino-2 phénylthio)-3 propionique correspondant,

d) on cyclise par chauffage à une température allant de 100 à 140 °C dans un hydrocarbure aromatique choisi parmi le toluène, un xylène et l'éthylbenzène, l'acide thréo hydroxy-2 (alkoxy-4 phényl)-3 (amino-2 phénylthio)-3 propionique ainsi obtenu, ce qui fournit un dérivé cis(2S, 3S) de formule générale :

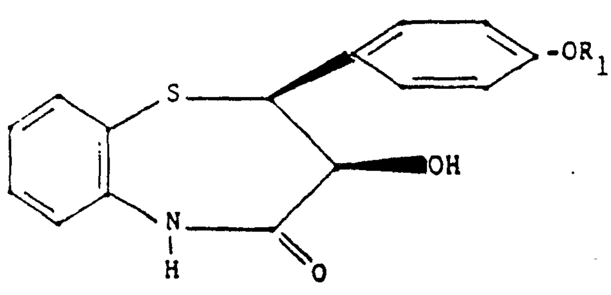

III

dans laquelle $R_1$ a la même signification que précédemment,

e) on fait réagir le dérivé cis(2S, 3S) de formule III avec un halogénure, de préférence le chlorure, de formule générale :

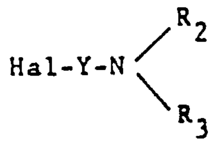

IV

dans laquelle Hal représente un atome de chlore, de brome ou d'iode et Y, $R_2$ et $R_3$ ont la même signification que précédemment, ou un sel de cet halogénure, en présence d'hydroxyde, de carbonate ou d'hydrogénocarbonate de potassium et dans un solvant choisi parmi le formamide, l'acétamide, le N,N-diméthylformamide et le N,N-diméthylacétamide pour obtenir undérivé cis(2S, 3S) de benzothiazépine-1,5 one-4 de formule générale :

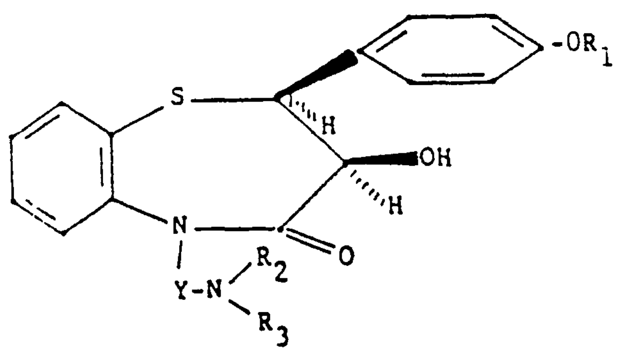

V

dans laquelle $R_1$, $R_2$, $R_3$ et Y ont la même signification que précédemment,

f) on fait réagir, dans un hydrocarbure aromatique au reflux, le dérivé cis(2S, 3S)de benzothiazépine-1,5 one-4 de formule V, avec un anhydride de formule générale

$R-\overset{\overset{O}{\|}}{C}-O-\overset{\overset{O}{\|}}{C}-R$ VI

dans laquelle R a la même signification que précédemment, pour obtenir le composé désiré de formule I sous forme de base libre que l'on peut faire réagir, si nécessaire, avec un acide organique ou inorganique approprié pour former des sels pharmaceutiquement acceptables.

2. Procédé selon la revendication 1, caractérisé en ce que R, $R_1$, $R_2$ et $R_3$ représentent chacun le radical méthyle et Y représente le radical éthylène.

3. Procédé selon la revendication 1 ou la revendication 2, caractérisé en ce que pour séparer les acides (+) et (-) hydroxy-2 (alkoxy-4 phényl)-3 (amino-2 phénylthio)-3 propioniques on soumet le mélange à une réaction de salification dans un solvant, avec la d- -phényléthylamine, on sépare les deux sels optiquement actifs par cristallisation fractionnée et on traite le sel du dérivé d'acide thréo hydroxy-2 (alkoxy-4 phényl)-3 (amino-2 phénylthio)-3 propionique de formule II et de la d-α-phényléthylamine avec un acide organique ou inorganique.

4. Procédé selon la revendication 3, caractérisé en ce que pour la salification de l'acide (+/-)thréo hydroxy-2 (alkoxy-4 phényl)-3 (amino-2 phénylthio)-3 propionique de formule II avec la d-α- phényléthylamine et la séparation des deux sels optiquement actifs, le solvant est l'eau.

5. Procédé selon une des revendications 1 à 4, caractérisé en ce que la cyclisation de l'acide thréo hydroxy-2 (alkoxy-4 phényl)-3 (amino-2 phénylthio)-3 propionique a lieu dans du xylène par chauffage au reflux.

6. Procédé selon une des revendictions 1 à 5 caractérisé en ce que la réaction du dérivé cis(2S,3S) de formule III avec l'halogénure de formule IV a lieu en présence de carbonate de potassium.

7. Procédé selon une des revendications 1 à 6, caractérisé en ce que la réaction du dérivé cis-(2S,3S) de formule III avec l'halogénure de formule IV a lieu dans le N,N-diméthylformamide comme solvant.

8. Procédé selon une des revendications 1 à 7, caractérisé en ce que la réaction du dérivé cis-(2S,3S) de formule III avec l'halogénure de formule IV a lieu à une température allant de la température ambiante à 80 °C.

9. Procédé selon une des revendications 1 à 8, caractérisé en ce que la réaction du dérivé cis-(2S,3S) de benzothiazépine-1,5 one-4 de formule V avec l'anhydride de formule VI a lieu dans le toluène comme solvant.

10. Procédé de préparation du cis(+)(2S, 3S) acétoxy-3 (N,N-diméthylamino-2 éthyl)-5 (méthoxy-4

phényl)-2 dihydro-2,3 benzothiazépine (5H)-1,5 one-4 de formule :

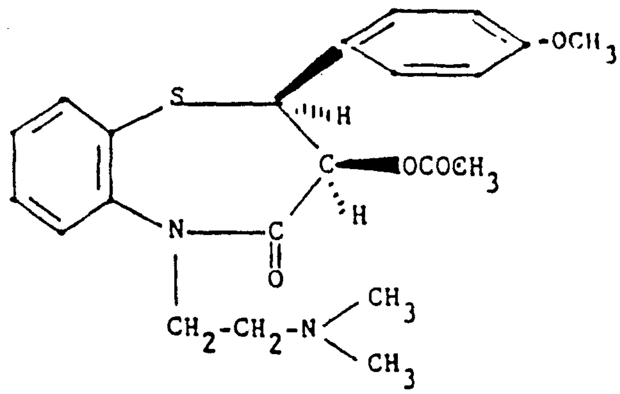

ainsi que de ses sels pharmaceutiquement acceptables, comprenant les étapes suivantes :

a) on fait réagir un ester glycidique trans (2RS, 3SR) de formule générale :

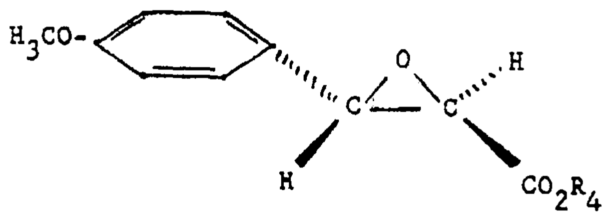

dans laquelle $R_4$ représente un radical alkyle en $C_1$-$C_4$, avec l'amino-2 thiophénol pour donner les composés threo (2RS, 3SR) de formule générale :

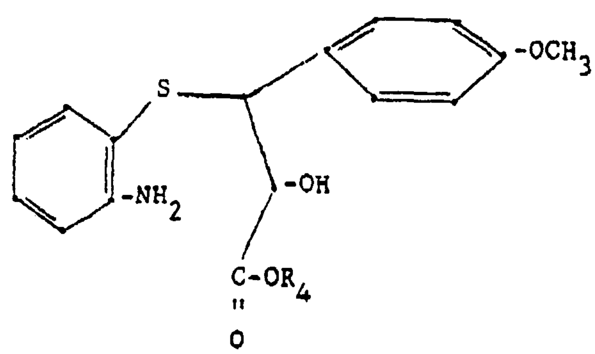

dans laquelle $R_4$ a la même signification que précédemment,

b) on saponifie le composé obtenu au moyen d'un hydroxyde de métal alcalin ou d'un carbonate de métal alcalin et on forme par acidification avec l'acide chlorhydrique, l'acide (+/-)threo hydroxy-2 (amino-2 phénylthio)-3(méthoxy-4 phényl)-3 propionique correspondant,

c) on soumet l'acide (+/-)thréo hydroxy-2 (amino-2 phénylthio)-3 (méthoxy-4 phényl)-3 propionique à une réaction de salification dans l'eau, avec la d-α-phényléthylamine, on sépare les deux sels optiquement actifs par cristallisation fractionnée et on traite le sel de l'acide thréo hydroxy-2 (méthoxy-4 phényl)-3 (amino-2 phénylthio)-3 propionique et de la d-α-phényléthylamine avec un acide organique ou inorganique pour obtenir l'acide (+)thréo hydroxy-2 (méthoxy-4 phényl)-3 (amino-2 phénylthio)-3 propionique

d) on cyclise dans du xylène par chauffage au reflux l'acide (+)thréo hydroxy-2 (méthoxy-4 phényl)-3 (amino-2 phénylthio)-3 propionique ainsi obtenu, ce qui, fournit la cis(2S, 3S) hydroxy-3 (méthoxy-4 phényl)-2 dihydro-2,3 benzothiazépine (5H)-1,5 one-4

e) on fait réagir la cis(+)(2S, 3S) hydroxy-3 (méthoxy-4 phényl)-2 dihydro-2,3 benzothiazépine (5H)-1,5 one-4 avec le diméthylamino-2 chloro-1 éthane ou son chlorhydrate en présence de carbonate de

potassium et dans le N,N-diméthylformamide comme solvant pour obtenir la cis(+)(2S,3S) hydroxy-3 (N,N-diméthylaminoéthyl)-5 (méthoxy-4 phényl)-2 dihydro-2,3 benzothiazépine (5H)-1,5 one-4

f) on fait réagir dans un hydrocarbure aromatique en chauffant au reflux, la cis(+)(2S,3S) hydroxy-3 (N,N-diméthylaminoéthyl)-5 (méthoxy-4 phényl)-2 dihydroxy-2,3 benzothiazépine (5H)-1,5 one-4 avec l'anhydride acétique, ce qui fournit le dérivé de benzothiazépine-1,5 one-4 désiré que l'on peut, si nécessaire, faire réagir avec un acide organique ou inorganique approprié pour former un sel pharmaceutiquement acceptable.

11. Procédé selon l'une des revendications 1 à 10, caractérisé en ce que dans l'étape e) on utilise 1 à 3 moles d'hydroxyde, de carbonate ou d'hydrogénocarbonate de potassium par mole de composé de formule III.

Office européen des brevets

# RAPPORT DE RECHERCHE EUROPEENNE

Numero de la demande

EP 90 40 0242

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | EP-A-0 158 339 (TANABE SEIYAKU)<br>* En entier; en particulier page 7, lignes 21-26; page 8, lignes 1-9; pages 31,32; exemple 6 *<br>--- | 1-11 | C 07 D 281/10 //<br>C 07 B 57/00<br>C 07 C 323/56 |
| D,Y | FR-A-2 530 243 (TANABE SEIYAKU)<br>* En entier *<br>--- | 1-11 | |
| Y | CHEMICAL ABSTRACTS, vol. 101, no. 21, 19 novembre 1984, page 700, résumé no. 191169y, Columbus, Ohio, US; & JP-A-59 16 861 (MITSUI TOATSU CHEMICALS INC.) 28-01-1984<br>* Résumé *<br>--- | 1-11 | |
| D,Y | CHEMICAL ABSTRACTS, vol. 90, no. 1, 1 janvier 1979, page 591, résumé no. 6431w, Columbus, Ohio, US; JP-A-78 18 038 (TANABE SEIYAKU CO., LTD) 13-06-1978<br>* Résumé *<br>----- | 1-11 | |

| DOMAINES TECHNIQUES RECHERCHES (Int. Cl.5) |
|---|
| C 07 D 281/00 |

Le présent rapport a été établi pour toutes les revendications

| Lieu de la recherche | Date d'achèvement de la recherche | Examinateur |
|---|---|---|
| LA HAYE | 12-04-1990 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant